(19) Europäisches Patentamt
European Patent Office
Office européen des brevets

(11) Veröffentlichungsnummer: **0 024 259**
**B1**

# (12) EUROPÄISCHE PATENTSCHRIFT

(45) Veröffentlichungstag der Patentschrift: **31.07.85**

(51) Int. Cl.[4]: **C 07 D 213/64,** A 01 N 43/40

(21) Anmeldenummer: **80810248.7**

(22) Anmeldetag: **08.08.80**

(54) **Derivate der 5-(Pyridyl-2-oxy)-2-nitrobenzoesäure, deren Herstellung und sie enthaltende herbizide Mittel.**

(30) Priorität: **14.08.79 CH 7430/79**

(43) Veröffentlichungstag der Anmeldung: **25.02.81 Patentblatt 81/8**

(45) Bekanntmachung des Hinweises auf die Patenterteilung: **31.07.85 Patentblatt 85/31**

(84) Benannte Vertragsstaaten: **BE CH DE FR GB IT LI NL**

(56) Entgegenhaltungen:
**DE - A - 2 753 900**
**FR - A - 2 428 031**
**US - A - 3 784 635**
**US - A - 3 928 416**

(73) Patentinhaber: **CIBA-GEIGY AG, Postfach, CH-4002 Basel (CH)**

(72) Erfinder: **Rempfler, Hermann, Dr., Brücklismattstrasse 16, CH-4107 Ettingen (CH)**

**EP 0 024 259 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Derivate der 5-(Pyridyl-2-oxy)-2-nitrobenzoesäure mit herbizider Wirkung, ferner die Herstellung dieser neuen Säurederivate, solche Verbindungen als Wirkstoff enthaltende Mittel, sowie deren Verwendung als Herbizide, vor allem als selektive Herbizide in Kulturen von Nutzpflanzen.

Die Derivate der 5-(Pyridyl-2-oxy)-2-nitrobenzoesäure sind neue Verbindungen. Aus der US-PS 3 928 416 und der DE-OS 2 753 900 sind 3-(para-Trifluormethylphenoxy)-6-nitrobenzoesäure-Derivate mit herbizider Wirkung bekannt geworden. In der US-PS 4 031 131 sind herbizide 3-(para-Trifluormethylphenoxy)-benzoesäuren und Derivate beschrieben worden und schließlich sind aus der US-PS 3 784 635 herbizide 3-(Phenoxy)-6-nitrobenzoesäurederivate bekannt geworden. Ferner sind in der europäischen Patentanmeldung 21 613 und der DE-OS 2 923 371 5-Pyridyloxy-2-nitrobenzoesäure-Derivate beschrieben.

Die Derivate der 5-(Pyridyl-2-oxy)-2-nitrobenzoesäure entsprechen der Formel I

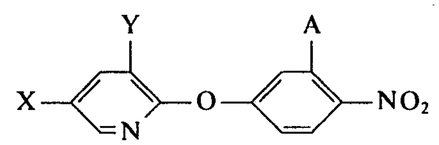

(I)

worin X Halogen oder eine Halogenmethylgruppe, Y Wasserstoff, Halogen oder eine Halogenmethylgruppe, A die Cyanogruppe oder einen Rest $-COB$, B einen Rest $-OR_1$, $-SR_2$, $-NR_3R_4$ oder $-ON{=}C(R_5)_2$, $R_1$ Wasserstoff oder das Kation einer Base $\left[\frac{1}{n} M\right]^{n\oplus}$, wobei M ein Alkali-, Erdalkali-Kation oder ein Fe-, Cu-, Zn- oder

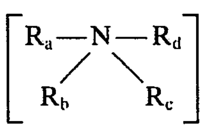

$^{\oplus}$-Kation,

n als ganze Zahl 1, 2 oder 3 die Wertigkeit des Kations berücksichtigt, während $R_a$, $R_b$, $R_c$ und unabhängig voneinander Wasserstoff, Benzyl oder einen gegebenenfalls durch $-OH$, $-NH_2$ oder $C_1$—$C_4$ Alkoxy substituierten $C_1$—$C_4$ Alkylrest bedeuten, $R_1$ und $R_2$ einen $C_1$—$C_{18}$ Alkylrest, der unsubstituiert oder gegebenenfalls substituiert ist durch Halogen, Nitro, Cyan, $C_1$—$C_8$ Alkoxy, $C_2$—$C_8$ Alkoxyalkoxy, Oxiran, $C_3$—$C_6$ Alkenyloxy, $C_1$—$C_8$ Alkylthio, $C_2$—$C_8$ Alkanoyl, $C_2$—$C_8$ Acyloxy, $C_2$—$C_8$ Alkoxycarbonyl, Carbamoyl, $C_1$—$C_4$ Alkylamino, Bis($C_1$—$C_4$ Alkyl)-amino, $C_3$—$C_4$ Alkenylamino, Bis($C_3$—$C_4$ Alkenyl)-amino, $C_3$—$C_4$ Alkinylamino, Bis($C_3$—$C_4$ Alkinyl)-amino, Tri($C_1$—$C_4$ Alkyl)-amino, $C_3$—$C_8$ Cycloalkyl, $C_3$—$C_8$ Cycloalkenyl, gegebenenfalls auch durch einen unsubstituierten oder durch Halogen, $C_1$—$C_4$ Alkyl, $C_1$—$C_4$ Alkoxy ein- oder mehrfach substituierten Phenyl-, Phenoxy- oder 5-6-gliedrigen heterocyclischen Rest mit 1 bis 3 Heteroatomen aus der Gruppe Sauerstoff, Schwefel und/oder Stickstoff; einen unsubstituierten oder ein- bis vierfach durch Halogen oder einen durch Phenyl oder Methoxycarbonyl substituierten $C_3$—$C_{10}$ Alkenylrest, einen $C_3$—$C_8$ Alkinyl-Rest, einen gegebenenfalls durch Halogen oder $C_1$—$C_4$ Alkyl substituierten $C_3$—$C_{12}$ Cycloalkyl-Rest, einen $C_3$—$C_8$ Cycloalkylen-Rest, einen Phenylrest, der unsubstituiert oder durch Halogen, $C_1$—$C_4$ Alkyl, $C_1$—$C_4$ Alkoxy, $C_1$—$C_4$ Alkylthio, $-NO_2$, $-CF_3$, $-COOH$, $-CN$, $-OH$, $-SO_3H$, $-NH_2$ oder $-NH(C_1$—$C_4$ Alkyl) oder $-(C_1$—$C_4$ Alkyl$)_2$ ein- oder mehrfach substituiert ist, einen 5- bis 6-gliedrigen heterocyclischen Ring mit 1 bis 3 Heteroatomen aus der Gruppe Sauerstoff, Schwefel und/oder Stickstoff, $R_3$ und $R_4$ je Wasserstoff, einen $C_1$—$C_8$ Alkylrest, gegebenenfalls substituiert durch $C_1$—$C_4$ Alkoxy, Hydroxyl, Halogen oder $C_2$—$C_8$ Alkoxycarbonyl, einen $C_3$—$C_5$ Alkenylrest, einen $C_3$—$C_5$ Alkinylrest, einen $C_3$—$C_7$ Cycloalkyl-, einen Pyridyl- oder gegebenenfalls durch Chlor substituierten Phenylrest oder eines davon auch einen $C_1$—$C_4$ Alkoxy- oder $C_1$—$C_4$ Alkylsulfonylrest oder $R_3$ und $R_4$ zusammen, mit dem Stickstoffatom, an das sie gebunden sind ebenfalls einen 5—6-gliedrigen Heterocyclus, der noch ein Sauerstoff- oder Schwefelatom oder eine gegebenenfalls durch $C_1$—$C_3$ Alkyl substituierte Iminogruppe enthalten kann, und $R_5$ je $C_1$—$C_4$ Alkyl oder zusammen eine $C_3$—$C_5$ Alkylenkette bedeuten, mit der Maßgabe, daß, wenn X Fluor, Chlor, Brom, Jod, Difluor-, Chlordifluor- oder Trifluormethyl und zugleich Y Wasserstoff, Fluor, Chlor, Brom, Jod, Difluor-, Chlordifluor- oder Trifluormethyl bedeutet und mindestens einer der Substituenten X und Y für Difluor-, Chlordifluor- oder Trifluormethyl steht, A nicht für einen Rest $-COB$, in welchem B für $-OR_1$ oder $-NR_3R_4$ steht, worin $R_1$ Wasserstoff, Kation einer Base $\left[\frac{1}{n} M\right]^{n\oplus}$, wobei M und n die vorstehend angegebenen Bedeutungen haben, oder einen $C_1$—$C_{12}$ Alkylrest, welcher gegebenenfalls durch $C_1$—$C_8$ Alkoxy substituiert ist, und $R_3$ und $R_4$ je Wasserstoff, einen $C_1$—$C_8$ Alkylrest, einen $C_3$—$C_5$ Alkenylrest oder eines davon auch einen $C_1$—$C_4$ Alkylsulfonylrest bedeuten, steht.

In der Formel I ist unter Halogen Fluor, Chlor, Brom oder Jod zu verstehen.

Der Ausdruck Alkyl allein oder als Teil eines Substituenten umfaßt verzweigte oder unverzweigte Alkylgruppen. Beispiele sind Methyl, Äthyl, Propyl, Isopropyl, Butyl, Isobutyl, sec. Butyl, tert. Butyl sowie die höheren Homologen Amyl, Isoamyl, Hexyl, Heptyl, Octyl samt ihre Isomeren.

Die Alkenylreste und Alkinylreste umfassen ebenfalls verzweigte und unverzweigte Reste, welche die gegebene Anzahl Kohlenstoffatome enthalten und mindestens eine ungesättigte Bindung. Bevorzugte Alkenylreste sind die gegebenenfalls durch Halogen substituierten Allyl-, Methallyl- und n-Butenylreste; der bevorzugte Alkinylrest ist der Propinyl- oder Propargylrest.

Cycloalkylreste sind Cyclopropyl, Cyclobutyl, Cyclopentyl, Cyclohexyl und Cycloheptyl, Cycloalkenylreste entsprechen diesen Ringsystemen, enthalten aber noch, je nach Möglichkeit, eine oder mehrere Doppelbindungen.

Heterocyclische Ringe enthalten 1 bis 3 gleiche oder verschiedene Heteroatome aus der Gruppe O, S und/oder N. Dies sind 5- oder 6gliedrige Heterocyclen, die gesättigt, oder ungesättigt und gegebenenfalls, wie oben erläutert, substituiert sind. Als Beispiele, die keine Limitierung darstellen sollen, seien genannt: Furan, Nitrofuran, Bromfuran, Methylfuran, Thiophen, Chlorthiophen, Pyridin, 2,6-Dichlorpyridin, Pyrimidin, Pyridazin, Pyrazin, Piperidin, Methylpiperidin, Morpholin, Thiomorpholin, Tetrahydrofuran, Oxazol, Pyrazol, Pyrrol, Pyrrolin, Pyrrolidin, Thiazol, 2,3-Dihydro-4H-pyran, Pyran, Dioxan und 1,4-Oxathi-(2)-in.

Die Verbindungen der Formel I weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Soja, Baumwolle, Getreide, Reis, Mais und Zuckerrüben.

Bei genügend großer Aufwandmenge ist jedoch auch totalherbizide Wirkung vorhanden. Die Anwendung kann sowohl im Vorauflauf- wie im Nachauflaufverfahren vorgenommen werden. Dabei können die Aufwandmengen in weiten Grenzen schwanken, z. B. zwischen 0,1 bis 10 kg Wirkstoff pro Hektar, vorzugsweise werden jedoch 0,5 bis 5 kg Wirkstoff pro Hektar eingesetzt.

Sehr gute Wirkung gegen Unkräuter in Kulturen von Weizen, Reis und/oder Soja wurde mit denjenigen Verbindungen erzielt, die strukturmäßig den folgenden Gruppen angehören, wobei für die Gruppen Ib und Ic die für Formel I gegebene Maßgabe gilt:

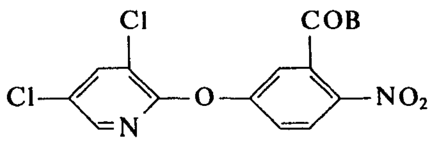

(Ia)

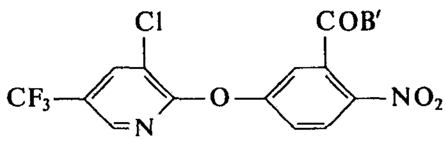

(Ib)

und

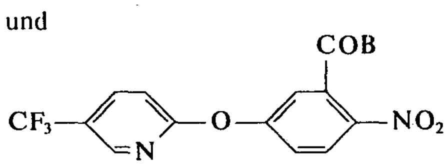

(Ic)

In diesen Verbindungen kann B generell jede unter Formel I definierte Bedeutung haben. Am wirksamsten waren Verbindungen, in denen B die Bedeutung Hydroxyl, $C_1$—$C_{18}$ Alkoxy, $C_1$—$C_4$ Cyanalkoxy, $C_3$— $C_{18}$ Alkoxycarbonylalkoxy, Di-($C_1$—$C_4$ alkyl)-amino-$C_1$—$C_4$-alkoxy, $C_3$—$C_{10}$ Alkenyloxy, $C_3$—$C_8$ Alkinyloxy, $C_1$—$C_{18}$ Alkylthio, $C_3$—$C_{18}$ Alkoxycarbonylalkylthio, $C_3$—$C_{10}$ Alkenylthio, $C_1$—$C_4$ Alkylamino, $C_2$—$C_8$ Alkoxyalkylamino, $C_3$—$C_8$ Alkoxycarbonylalkylamino, N-$C_1$—$C_4$-Alkoxy-N-$C_1$—$C_4$-alkylamino hat.

Zu erwähnen sind ferner Verbindungen der Formel Ib, in welchen B' einen Rest $-OR_1$, $-SR_2$, $-NR_3R_4$ oder $-ON{=}C(R_5)_2$ bedeutet, worin $R_1$ einen substituierten $C_1$—$C_{18}$ Alkylrest, einen $C_3$—$C_{10}$ Alkenylrest, einen $C_3$—$C_8$ Alkinylrest, einen $C_3$—$C_{12}$ Cycloalkylrest, einen unsubstituierten oder durch Halogen, $C_1$—$C_4$ Alkyl, $C_1$—$C_4$ Alkoxy, $C_1$—$C_4$ Alkylthio, $-NO_2$, $-CF_3$, $-COOH$, $-CN$, $-OH$, $-SO_3H$, $-NH(C_1$—$C_4$ Alkyl) oder $-N(C_1$—$C_4$ $Alkyl)_2$ ein- oder mehrfach substituierten Phenylrest bedeutet und $R_2$, $R_3$, $R_4$ und $R_5$ die für Formel I angegebenen Bedeutungen haben.

Erfindungsgemäße Mittel enthalten außer dem Wirkstoff der Formel I einen geeigneten Träger und/oder andere Zuschlagstoffe. Geeignete Träger und Zuschlagstoffe können fest oder flüssig sein und entsprechen den in der Formulierungstechnik üblichen Stoffen wie z. B. natürlichen oder regenerierten mineralischen Stoffen, Lösungs-, Verdünnungs-, Dispergier-, Emulgier-, Netz-, Haft-, Verdickungs-, Binde- und/oder Düngemitteln.

Zur Verwendung in herbiziden Mitteln kann der Wirkstoff der Formel I als Stäubemittel, Emulsionskonzentrat, als Granulat, Dispersion oder auch als Lösung oder Aufschlämmung in üblicher Formulierung verarbeitet werden.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;

In Wasser dispergierbare Wirkstoffkonzentrate:
Spritzpulver (wettable powder), Pasten, Emulsionen, Emulsionskonzentrate.

Flüssige Aufarbeitungsformen:
Lösungen.

Die Wirkstoffkonzentrationen betragen in den erfindungsgemäßen Mitteln 1 bis 80 Gewichtsprozent und können gegebenenfalls bei der Anwendung auch in geringeren Konzentrationen wie etwa 0,05 bis 1 % vorliegen.

Den erfindungsgemäßen Mitteln lassen sich noch andere biozide Wirkstoffe oder Mittel beimischen. So können die neuen Mittel außer den genannten Verbindungen der Formel I z. B. Insektizide, Fungizide, Bakterizide, Fungistatika, Bakteriostatika, Nematozide oder weitere Herbizide zur Verbreiterung des Wirkungsspektrums enthalten.

Die Herstellung der Verbindungen der Formel I erfolgt nach an sich bekannten Methoden, zum Beispiel über einen der nachfolgend beschriebenen Synthesewege:

Gemäß einem ersten Verfahren zur Herstellung der 5-(Pyridyl-2-oxy)-2-nitrobenzoesäurederivate der Formel I, setzt man in einem aprotischen Lösungsmittel, in Gegenwart der säurebindenden Menge einer Base, ein 2-Halogenpyridin der Formel II

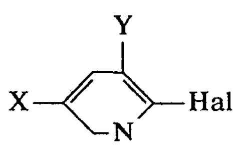 (II)

worin Hal Halogen bedeutet und X und Y die gegebene Bedeutung haben, mit einem Derivat der 3-Hydroxy-6-nitrobenzoesäure der Formel III um

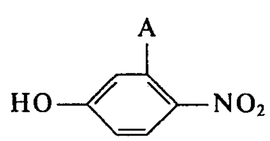 (III)

worin A einen bezüglich Formel I definierten Rest darstellt.

Gemäß einem weiteren Syntheseweg wird das 2-Halogenpyridin der Formel II und ein 3-Hydroxybenzoesäurederivat der Formel IV

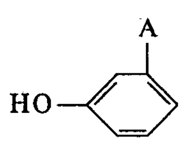 (IV)

worin A einen bezüglich Formel I definierten Rest darstellt, zuerst in einem aprotischen Lösungsmittel, in Gegenwart der säurebindenden Menge einer Base, zum 3-(Pyridyl-2-oxy)-benzoesäurederivat der Formel V umgesetzt

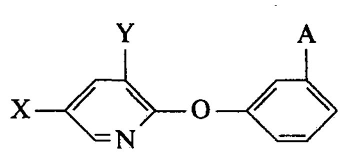 (V)

worin A, X und Y die gegebene Bedeutung haben.

Schließlich wird das 3-(Pyridyl)-2-oxy)-benzoesäurederivat der Formel V mit einem Salpetersäuregemisch nitriert.

Schließlich können in Verbindungen der Formel I, in denen A beispielsweise das Säurenitril, die Carboxylgruppe oder das Carbonsäureamid darstellt, diese Reste durch geeignete Umsetzungen, wie Verseifen, Verestern, Aminieren oder Alkylieren, in andere unter die Definition von A fallende Carbonsäurederivate umgewandelt werden.

Diese Umsetzungen werden bei Temperaturen von 0 bis 150°C durchgeführt. Als Lösungsmittel eignen sich vor allem aprotische, wie Dimethylsulfoxyd, Dimethylformamid, Sulfolan oder N-Methylpyrrolidon.

Als Basen, die zur Bindung der abgespaltenen Halogenwasserstoffsäure zugegeben werden, eignen sich sowohl anorganische, wie Alkalimetallhydroxyde, Alkali- sowie Erdalkalimetall-carbonate und -bicarbonate wie auch organische Basen, wie z. B. die Alkylamine Dimethylamin, Trimethylamin, Diäthylamin oder Triäthylamin.

Als Nitrierlösungen kommen beispielsweise Mischungen von Salpeter- und Schwefelsäure, Lösungen von Salpetersäure in Eisessig oder konzentrierte Salpetersäure in chlorierten Kohlenwasserstoffen in Frage. Die Nitrierung wird bei Temperaturen um 0°C bis Raumtemperatur vorgenommen.

Die Verbindungen der Formel I sind stabile Verbindungen, für Warmblüter wenig giftig und ihre Handhabung bedarf keiner vorsorglichen Maßnahme. Sie sind in den üblichen organischen Lösungsmitteln relativ gut, in Wasser jedoch schlecht löslich.

In den folgenden Beispielen wird die Herstellung der erfindungsgemäßen 5-(Pyridyl-2-oxy)-2-nitrobenzoesäuren der Formel I näher erläutert. Weitere in analoger Weise hergestellte Verbindungen sind in der anschließenden Tabelle aufgeführt. Die Temperaturen sind in Celsiusgraden angegeben, Teile und Prozentangaben beziehen sich auf Gewicht. Druckangaben werden in Millibar (mbar) gegeben.

## Beispiel 1

2-Nitro-5-(3,5-dichlorpyridyl-2-oxy)-2-benzonitril

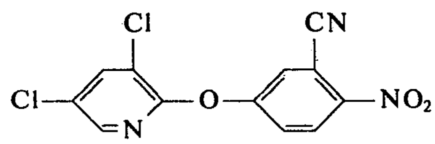

Zu einer Suspension von 2,4 g Natriumhydrid (0,1 Mol) in 30 ml N-Methylpyrrolidon werden 16,4 g 3-Cyano-4-nitrophenol (0,1 Mol) in 50 ml N-Methylpyrrolidon gelöst, zugetropft. Nach beendeter Wasserstoffentwicklung werden 16,6 g 2-Fluor-3,5-dichlorpyridin (0,1 Mol) zugegeben. Das Reaktionsgemisch wird 15 Stunden bei 110—115°C gerührt und dann auf Eiswasser gegossen. Das Wasser wird mit Chloroform extrahiert, das Chloroform über Natriumsulfat getrocknet und abgedampft. Der Rückstand wird auf einer Kieselgelsäule mit Essigester/Hexan gereinigt. Man erhält 6,2 g (30% der Theorie) der Titelverbindung mit einem Schmelzpunkt von 138—140°C.

## Beispiel 2

2-Nitro-5-(3,5-dichlorpyridyl-2-oxy)-benzoesäuremethylester

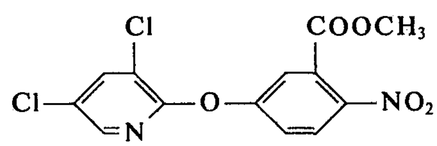

6,6 g 2-Nitro-5-(3,5-dichlorpyridyl-2-oxy)-benzoesäure, hergestellt analog Beispiel 1, werden mit 10 ml Thionylchlorid vermischt und während 15 Stunden bei 60°C gerührt. Das überschüssige Thionylchlorid wird abgedampft. Der Rückstand wird in 50 ml Toluol gelöst. Zu dieser Lösung werden 1 g Methanol und 2,1 g Triäthylamin zugegeben. Nach 1 Stunde wird das Salz abfiltriert, das Filtrat eingedampft. Der Rückstand kristallisiert bei der Zugabe von Essigester und Hexan aus. Man erhält 4 g (62% der Theorie) der Titelverbindung mit einem Schmelzpunkt von 81—82°C.

In analoger Weise zu diesen Beispielen wurden folgende Verbindungen erhalten:

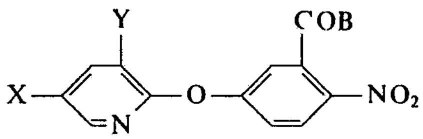

| No. | X | Y | B | Physikalische Daten |
|---|---|---|---|---|
| 1 | Cl | Cl | OH | Smp. 166–169° |
| 2 | Cl | Cl | $OCH_3$ | Smp. 81–82° |
| 3 | Cl | Cl | $OC_2H_5$ | |
| 4 | Cl | Cl | $OC_3H_7$n | |
| 5 | Cl | Cl | $OC_3H_7$iso | $n_D^{25}$ 1,5815 |
| 6 | Cl | Cl | $OC_4H_9$n | |
| 7 | Cl | Cl | $OC_4H_9$sec. | $n_D^{30}$ 1,5840 |
| 8 | Cl | Cl | $OC_4H_9$iso | $n_D^{25}$ 1,5680 |
| 9 | Cl | Cl | $OC_4H_9$tert. | |
| 10 | Cl | Cl | $OC_5H_{11}$iso | $n_D^{27}$ 1,5675 |
| 11 | Cl | Cl | $CH_3$<br>\|<br>$OCHC_3H_7$ | $n_D^{27}$ 1,5677 |
| 12 | Cl | Cl | $OCH(C_2H_5)_2$ | |
| 13 | Cl | Cl | $OCH_2CH_2OCH_3$ | |
| 14 | Cl | Cl | $OCH_2CH_2OC_2H_5$ | |
| 15 | Cl | Cl | $OCH_2CH_2OC_4H_9$ | $n_D^{27}$ 1,5620 |
| 16 | Cl | Cl | 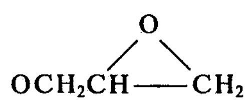 | |
| 17 | Cl | Cl | $OCH_2CH_2N(CH_3)_2$ | $n_D^{30}$ 1,5817 |
| 18 | Cl | Cl | 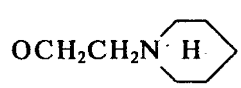 | |
| 19 | Cl | Cl | $CH_3$<br>\|<br>$OCHCOOCH_3$ | $n_D^{27}$ 1,5605 |
| 20 | Cl | Cl | $O(CH_2)_3N(CH_3)_2$ | |
| 21 | Cl | Cl | $OCH_2CH{=}CH_2$ | |
| 22 | Cl | Cl | $OCH_2CH{=}CH{-}CH_3$ | $n_D^{25}$ 1,5877 |

Fortsetzung

| No. | X | Y | B | Physikalische Daten |
|---|---|---|---|---|
| 23 | Cl | Cl | $OCH_2C{\equiv}CH$ | Smp. 81–82° |
| 24 | Cl | Cl | $OCH_2C{\equiv}CH_3$ | |
| 25 | Cl | Cl | 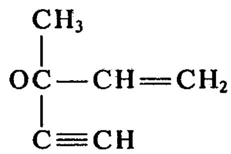 | |
| 26 | Cl | Cl | 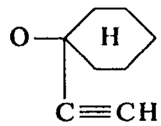 | |
| 27 | Cl | Cl | $OCH_2CN$ | $n_D^{27}$ 1,5890 |
| 28 | Cl | Cl | $OCH_2CH_2Cl$ | |
| 29 | Cl | Cl | $OCH_2CH_2Br$ | |
| 30 | Cl | Cl | $NH_2$ | Smp. 179–180° |
| 31 | Cl | Cl | $NHCH_3$ | |
| 32 | Cl | Cl | $NHC_2H_5$ | Smp. 119–120° |
| 33 | Cl | Cl | $NHC_3H_7$iso | |
| 34 | Cl | Cl | $NHCH_2CH{=}CH_2$ | |
| 35 | Cl | Cl | $NHCH_2C{\equiv}CH$ | |
| 36 | Cl | Cl | $NHC_4H_9$sec | |
| 37 | Cl | Cl | $NHCH(C_2H_5)_2$ | |
| 38 | Cl | Cl | $NHCH_2COOCH_3$ | |
| 39 | Cl | Cl | $N(CH_3)_2$ | |
| 40 | Cl | Cl | $N(C_2H_5)_2$ | |
| 41 | Cl | Cl | 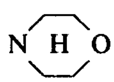 | |
| 42 | Cl | Cl | 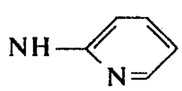 | |
| 43 | Cl | H | OH | Smp. 166–169° |
| 44 | Cl | H | $OCH_3$ | |
| 45 | Cl | H | $OC_2H_5$ | |

Fortsetzung

| No. | X | Y | B | Physikalische Daten |
|---|---|---|---|---|
| 46 | Cl | H | $N(CH_3)_2$ | |
| 47 | Cl | H | $NHCH_2COOC_2H_5$ | |
| 48 | Cl | Cl | NH— 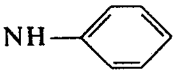 | |
| 49 | Cl | Cl | $SCH_3$ | |
| 50 | Cl | Cl | $SC_2H_5$ | $n_D^{27}$ 1,6200 |
| 51 | Cl | Cl | $SC_4H_9$iso | $n_D^{27}$ 1,5965 |
| 52 | Cl | Cl | $SCH_2CH{=}CH_2$ | |
| 53 | Cl | Cl | $SCH_2COOCH_3$ | $n_D^{27}$ 1,6060 |
| 54 | Cl | Cl | $SCH_2C{\equiv}CH$ | |
| 55 | Cl | Br | OH | |
| 56 | Cl | Br | $OCH_3$ | |
| 57 | Cl | Br | $OC_2H_5$ | |
| 58 | Cl | Br | $OC_5H_9$iso | |
| 59 | Cl | Br | $OC_4H_9$sec | |
| 60 | Cl | Br | $SC_3H_7$n | |
| 61 | Cl, | Br | $SC_4H_9$n | |
| 62 | Cl | Br | $SCH_2CH{=}CH_2$ | |
| 63 | Cl | Br | $OCH(CH_2)_4$ | |
| 64 | Cl | Br | $OCH_2CH(CH_2)_5$ | |
| 65 | Cl | Br | $NHC_2H_5$ | |
| 66 | Cl | Br | $NHC_3H_7$iso | |
| 67 | Cl | Br | $NHC_8H_{17}$ | |
| 68 | Cl | Br | $N(CH_2CH{=}CH_2)_2$ | |
| 69 | Br | Cl | OH | |
| 70 | Br | Cl | $OCH_3$ | |
| 71 | Br | Cl | $OC_2H_5$ | |
| 72 | Br | Cl | $OC_3H_7$iso | |

Fortsetzung

| No. | X | Y | B | Physikalische Daten |
|---|---|---|---|---|
| 73 | Br | Cl | $OC_4H_9$sec | |
| 74 | Br | Cl | $OC_4H_9$iso | |
| 75 | Br | Cl | $OC_4H_9$tert. | |
| 76 | Br | Cl | $OCH(CH_2CH_3)_2$ | |
| 77 | Br | Cl | 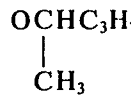 | |
| 78 | Br | Cl | $SCH_3$ | |
| 79 | Br | Cl | $SC_3H_7$ | |
| 80 | $CF_3$ | Cl | $O(CH_2)_4Cl$ | $n_D^{30}$ 1,5350 |
| 81 | $CF_3$ | Cl | $OCH_2CH_2Cl$ | $n_D^{30}$ 1,5485 |
| 82 | $CF_3$ | Cl | $OCH_2CH_2Br$ | |
| 83 | $CF_3$ | Cl | $OCH_2CH_2F$ | |
| 84 | $CF_3$ | Cl | $OCH_2CN$ | Smp. 100–102° |
| 85 | $CF_3$ | Cl | 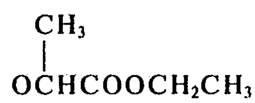 | $n_D^{30}$ 1,5213 |
| 86 | $CF_3$ | Cl | $OCH_2CH{=}CH_2$ | $n_D^{30}$ 1,5411 |
| 87 | $CF_3$ | Cl | $OCH_2CH{=}CHCH_3$ | |
| 88 | $CF_3$ | Cl | $OCH_2C{\equiv}CH$ | Smp. 94–96° |
| 89 | $CF_3$ | Cl | 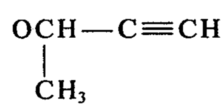 | |
| 90 | $CF_3$ | Cl | 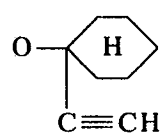 | |
| 91 | $CF_3$ | Cl | 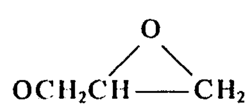 | |
| 92 | $CF_3$ | Cl | 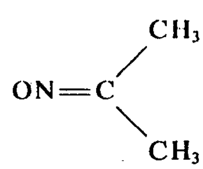 | Smp. 89–91° |

Fortsetzung

| No. | X | Y | B | Physikalische Daten |
|---|---|---|---|---|
| 93 | $CF_3$ | Cl | 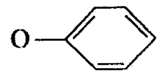 | |
| 94 | $CF_3$ | Cl | 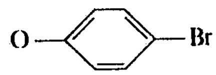 | Smp. 92–94° |
| 95 | $CF_3$ | Cl | 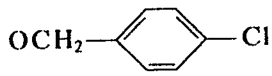 | Smp. 79–81° |
| 96 | $CF_3$ | Cl | $OCH_2CH_2N(CH_3)_2$ | $n_D^{30}$ 1,5336 |
| 97 | $CF_3$ | Cl | 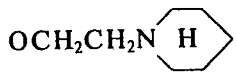 | |
| 98 | $CF_3$ | Cl | 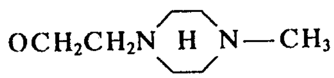 | |
| 99 | $CF_3$ | Cl | $SCH_3$ | $n_D^{30}$ 1,5695 |
| 100 | $CF_3$ | Cl | $SC_2H_5$ | |
| 101 | $CF_3$ | Cl | $SC_3H_7n$ | |
| 102 | $CF_3$ | Cl | $SC_3H_7iso$ | $n_D^{30}$ 1,5590 |
| 103 | $CF_3$ | Cl | $SC_4H_9n$ | |
| 104 | $CF_3$ | Cl | $SC_4H_9iso$ | |
| 105 | $CF_3$ | Cl | $SC_5H_{11}n$ | |
| 106 | $CF_3$ | Cl | $SCH_2COOCH_3$ | $n_D^{30}$ 1,5650 |
| 107 | $CF_3$ | Cl | $SCH_2{=}CH_2$ | $n_D^{30}$ 1,5725 |
| 108 | $CF_3$ | Cl | $SCH_2C{\equiv}CH$ | |
| 109 | $CF_3$ | Cl | $SCH_2CH{=}CHCH_3$ | |
| 110 | $CF_3$ | Cl | $SCH_2C(CH_3){=}CH_2$ | |
| 111 | $CF_3$ | Cl | 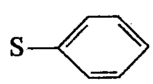 | |
| 112 | $CF_3$ | Cl | 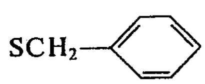 | $n_D^{30}$ 1,5972 |
| 113 | $CF_3$ | Cl | $NHCH_2CH_2Cl$ | |
| 114 | $CF_3$ | Cl | $NHCH_2CH_2OH$ | |
| 115 | $CF_3$ | Cl | $NHCH_2CH_2OCH_3$ | |

Fortsetzung

| No. | X | Y | B | Physikalische Daten |
|---|---|---|---|---|
| 116 | $CF_3$ | Cl | $NHCH_2CH_2OC_4H_9$ | |
| 117 | $CF_3$ | Cl | $NHCH_2COOC_2H_5$ | |
| 118 | $CF_3$ | Cl | $NHCH_2C{\equiv}CH$ | |
| 119 | $CF_3$ | Cl | $NHC(CH_3)_2CN$ | |
| 120 | $CF_3$ | Cl | NH 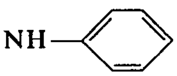 | |
| 121 | $CF_3$ | Cl | 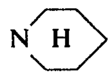 | |
| 122 | $CF_3$ | Cl | 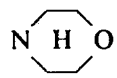 | |
| 123 | $CF_3$ | Cl | $S^{\ominus}K^{\oplus}$ | |
| 124 | $CF_3$ | Cl | $S^{\ominus}Na^{\oplus}$ | |
| 125 | $CF_3$ | H | $SCH_3$ | |
| 126 | $CF_3$ | H | $SC_2H_5$ | |
| 127 | $CF_3$ | H | $SC_3H_7$iso | |
| 128 | $CF_3$ | H | $SC_4H_9$iso | |
| 129 | $CF_3$ | H | $SC_5H_{11}$ | |
| 130 | $CF_3$ | H | $NHCH_2CH_2Cl$ | |
| 131 | $CF_3$ | H | $NHCH_2C{\equiv}CH$ | |
| 132 | $CF_3$ | H | $NHCH_2COOCH_3$ | |
| 133 | $CF_3$ | H | NH 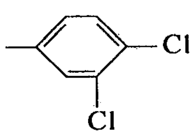 | |
| 134 | Cl | $CF_3$ | $SCH_3$ | |
| 135 | $CCl_2F$ | Cl | OH | |
| 136 | $CCl_2F$ | Cl | $OCH_3$ | |
| 137 | $CCl_2F$ | Cl | $OC_2H_5$ | |
| 138 | $CCl_2F$ | Cl | $OC_3H_7$n | |
| 139 | $CCl_2F$ | Cl | $OC_3H_7$iso | |
| 140 | $CCl_2F$ | Cl | $OC_4H_9$iso | |

Fortsetzung

| No. | X | Y | B | Physikalische Daten |
|---|---|---|---|---|
| 141 | $CCl_2F$ | Cl | $OC_4H_9sec$ | |
| 142 | $CCl_2F$ | Cl | $OCH_2CH_2OCH_3$ | |
| 143 | $CCl_2F$ | Cl | $SCH_3$ | |
| 144 | $CCl_2F$ | Cl | $SC_2H_5$ | |
| 145 | $CCl_2F$ | Cl | $SCH_2COOCH_3$ | |
| 146 | $CCl_2F$ | Cl | $NHCH_3$ | |
| 147 | $CCl_2F$ | Cl | $N(CH_3)_2$ | |
| 148 | $CClF_2$ | Cl | $SCH_3$ | |
| 149 | $CF_3$ | Br | $SCH_3$ | |
| 150 | $CF_3$ | Br | $SC_3H_7iso$ | |
| 151 | $CF_3$ | Br | $NHCH_2COOCH_3$ | |
| 152 | $CF_3$ | Br | $OCH_2CH{=}CH_2$ | |
| 153 | Cl | Cl | $NHC(CH_3)_2CN$ | Smp. 160–162° |
| 154 | Cl | Cl | $NHCH_2CH_2OCH_3$ | Smp. 130–135° |
| 155 | Cl | Cl | 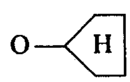 | Smp. 96–99° |
| 156 | Cl | Cl | $OCH(CH_3)C_5H_{11}$ | $n_D^{27}$ 1,5600 |
| 157 | Cl | Cl | $N(CH_3)OCH_3$ | $n_D^{34}$ 1,6055 |
| 158 | Cl | Cl | $OCH(CH_3)COOCH_2CH_3$ | |
| 159 | Cl | Cl | $SC_4H_9sec$ | $n_D^{27}$ 1,6000 |
| 160 | $CF_3$ | Cl | $SCH_2COOCH_2CH_3$ | |
| 161 | $CF_3$ | Cl | $N(CH_3)OCH_3$ | $n_D^{30}$ 1,5438 |

## Beispiel 3

Herstellung einiger gebrauchsfertiger Anwendungsformen und Wirkstoffkonzentrate:

### Paste

Zur Herstellung einer 45%igen Paste werden folgende Stoffe verwendet:

45 Teile 2-Nitro-5-(3,5-dichlorpyridyl-2-oxy)benzoesäure,
5 Teile Natriumaluminiumsilikat,

14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol,
23 Teile Wasser.

Der Wirkstoff wird mit den Zuschlagsstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%iges Emulsionskonzentrates werden

25 Teile 2-Nitro-5-(3,5-dichlorpyridyl-2-oxy)-benzoesäuremethylester,
10 Teile einer Mischung von Nonylphenolpolyoxyäthylen oder Calciumdidecylbenzolsulfonat,
10 Teile Cyclohexanon,
55 Teile Xylol

miteinander vermischt. Dieses Konzentrat kann mit Wasser zu Emulsionen auf geeignete Konzentrationen von z. B. 0,1 % verdünnt werden. Solche Emulsionen eignen sich zur Bekämpfung von Unkräutern in Kulturpflanzungen.

Spritzpulver

Zur Herstellung eines a) 70%igen und b) 10%igen Spritzpulvers werden folgende Bestandteile verwendet:

a) 70 Teile 2-Nitro-5-(3,5-dichlorpyridyl-2-oxy)-benzoesäureisopropylester,
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 10 Teile des obigen Wirkstoffes,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechende Trägerstoffe (Kaolin und Kreide) aufgezogen und anschließend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1–8% Wirkstoff erhalten werden, die sich zur Unkrautbekämpfung in Pflanzenkulturen eignen.

Beispiel 4

Die herbizide Wirkung der erfindungsgemäßen Verbindungen wurde in folgenden Gewächsversuchen ermittelt:

Herbizidwirkung im Vorauflaufversuch (Keimhemmung)

Im Gewächshaus werden Pflanzensamen in Blumentöpfe von ca. 15 cm Durchmesser gesät, so daß sich pro Topf 12–30 Pflänzchen entwickeln können. Unmittelbar nach der Saat der Versuchspflanzen wird die Erdoberfläche mit einer wäßrigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Blumentöpfe werden im Gewächshaus bei 22–25°C und 50–70% relativer Luftfeuchtigkeit gehalten und der Versuch nach 3 Wochen ausgewertet.

Die geprüften Verbindungen der Formel I zeigten in diesem Versuch bei Aufwandmengen von 1 und 2 kg/ha praktisch verwendbare Wirkung gegen die breitblättrigen und auch gegen die meisten grasartigen Unkräuter, während Kulturpflanzen wie Mais und zum Teil auch Weizen, Hirse, Reis, Soja und Baumwolle nicht oder nur unbedeutend geschädigt wurden.

Herbizidwirkung im Nachauflaufverfahren (Kontaktwirkung)

Eine größere Anzahl Unkräuter und Kulturpflanzen, sowohl monocotyle wie dicotyle, wurden nach dem Auflaufen (im 4- bis 6-Blattstadium) mit einer wäßrigen Wirkstoffdispersion in Dosierungen von 0,5, 1, 2 und 4 kg Wirksubstanz pro Hektar auf die Pflanzen gespritzt und diese bei 24—26°C und 45—60% relativer Luftfeuchtigkeit gehalten. 3 Wochen nach der Behandlung wurde der Versuch ausgewertet.

Auch in diesem Versuch zeigten die Verbindungen der Formel I eine durchaus brauchbare Wirkung gegen die breitblättrigen und die meisten der grasartigen Unkräuter bei der Aufwandmenge von 1 kg/ha, wobei die Kulturpflanze Mais, die Getreidearten Gerste, Hirse und Reis wie auch Baumwolle und Soja nicht oder erst mit höheren Aufwandmengen zu Wirkstoff geschädigt wurden.

**Patentansprüche**

1. Derivate der 5-(Pyridyl-2-oxy)-2-nitrobenzoesäure der Formel I

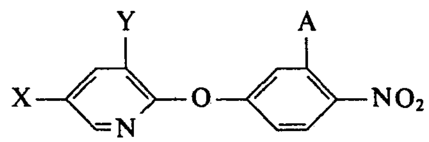

(I)

worin X Halogen oder eine Halogenmethylgruppe, Y Wasserstoff, Halogen, oder eine Halogenmethylgruppe, A die Cyanogruppe oder einen Rest $-COB$, B einen Rest $-OR_1$, $-SR_2$, $-NR_3R_4$ oder $-ON{=}C(R_5)_2$, $R_1$ Wasserstoff oder das Kation einer Base $\left[\frac{1}{n} M\right]^{n\oplus}$, wobei M ein Alkali-, Erdalkali-Kation oder ein Fe-, Cu-, Zn- oder

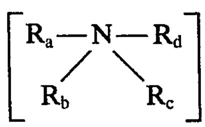

$^{\oplus}$-Kation

darstellt, n als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch $-OH$, $-NH_2$ oder $C_1$—$C_4$ Alkoxy substituierten $C_1$—$C_4$ Alkylrest bedeuten, $R_1$ und $R_2$ einen $C_1$—$C_{18}$ Alkylrest, der unsubstituiert oder gegebenenfalls substituiert ist durch Halogen, Oxiran, Nitro, Cyan, $C_1$—$C_8$ Alkoxy, $C_2$—$C_8$ Alkoxyalkoxy, $C_3$—$C_6$ Alkenyloxy, $C_1$—$C_8$ Alkylthio, $C_2$—$C_8$ Alkanoyl, $C_2$—$C_8$ Acyloxy, $C_2$—$C_8$ Alkoxycarbonyl, Carbamoyl, $C_1$—$C_4$ Alkylamino, Bis($C_1$—$C_4$ Alkyl)-amino, $C_3$—$C_4$ Alkenylamino, Bis($C_3$—$C_4$ Alkenyl)-amino, $C_3$—$C_4$ Alkinylamino, Bis($C_3$—$C_4$ Alkinyl)-amino, Tri($C_1$—$C_4$ Alkyl)-amino, $C_3$—$C_8$ Cycloalkyl, $C_3$—$C_8$ Cycloalkenyl, gegebenenfalls auch durch einen unsubstituierten oder durch Halogen, $C_1$—$C_4$ Alkyl, $C_1$—$C_4$ Alkoxy ein- oder mehrfach substituierten Phenyl-, Phenoxy- oder 5—6gliedrigen heterocyclischen Rest mit 1 bis 3 Heteroatomen aus der Gruppe Sauerstoff, Schwefel und/oder Stickstoff; einen unsubstituierten oder ein- bis vierfach durch Halogen oder einen durch Phenyl oder Methoxycarbonyl substituierten $C_3$—$C_{10}$ Alkenylrest, einen $C_3$—$C_8$ Alkinyl-Rest, einen gegebenenfalls durch Halogen oder $C_1$—$C_4$ Alkyl substituierten $C_3$—$C_{12}$ Cycloalkyl-Rest, einen $C_3$—$C_8$ Cycloalkylenrest, einen Phenylrest, der unsubstituiert oder durch Halogen, $C_1$—$C_4$ Alkyl, $C_1$—$C_4$ Alkoxy, $C_1$—$C_4$ Alkylthio, $-NO_2$, $-CF_3$, $-COOH$, $-CN$, $-OH$, $-SO_3H$, $-NH_2$ oder $-NH(C_1$—$C_4$ Alkyl) oder $-(C_1$—$C_4$ Alkyl$)_2$ ein- oder mehrfach substituiert ist, einen 5- bis 6gliedrigen heterocyclischen Ring mit 1 bis 3 Heteroatomen aus der Gruppe Sauerstoff, Schwefel und/oder Stickstoff, $R_3$ und $R_4$ je Wasserstoff, einen $C_1$—$C_8$ Alkylrest, der gegebenenfalls durch $C_1$—$C_4$ Alkoxy, Hydroxyl, Halogen oder $C_2$—$C_8$ Alkoxycarbonyl substituiert ist, einen $C_3$—$C_5$ Alkenylrest, einen $C_3$—$C_5$ Alkinylrest, einen $C_3$—$C_7$ Cycloalkylrest, einen Pyridylrest oder einen gegebenenfalls durch Chlor substituierten Phenylrest oder eines davon einen $C_1$—$C_4$ Alkoxy- oder $C_1$—$C_4$ Alkylsulfonylrest, $R_3$ und $R_4$ zusammen mit dem Stickstoffatom, an das sie gebunden sind, ebenfalls einen 5—6gliedrigen Heterocyclus, der noch ein Sauerstoff- oder Schwefelatom oder eine gegebenenfalls durch $C_1$—$C_3$ Alkyl substituierte Iminogruppe enthalten kann, und $R_5$ je $C_1$—$C_4$ Alkyl oder zusammen eine $C_3$—$C_5$ Alkylenkette bedeuten, mit der Maßgabe, daß, wenn X Fluor, Chlor, Brom, Jod, Difluor-, Chlordifluor- oder Trifluormethyl und zugleich Y Wasserstoff, Fluor, Chlor, Brom, Jod, Difluor-, Chlordifluor- oder Trifluormethyl bedeutet und mindestens einer der Substituenten X und Y für Difluor-, Chlordifluor- oder Trifluormethyl steht, A nicht für einen Rest $-COB$, in welchem B für $-OR_1$ oder

$-NR_3R_4$ steht, worin $R_1$ Wasserstoff, Kation einer Base $\left[\frac{1}{n} M\right]^{n(+)}$, wobei M und n die vorstehend angegebenen Bedeutungen haben, oder einen $C_1$—$C_{12}$ Alkylrest, welcher gegebenenfalls durch $C_1$—$C_8$ Alkoxy substituiert ist, und $R_3$ und $R_4$ je Wasserstoff, einen $C_1$—$C_8$ Alkylrest, einen $C_3$—$C_5$ Alkenylrest oder eines davon auch einen $C_1$—$C_4$ Alkylsulfonylrest bedeuten, steht.

2. Die 5-(Pyridyl-2-oxy)-2-nitro-benzoesäurederivate gemäß Anspruch 1, in denen A den COB-Rest verkörpert, während X, Y und B die in Anspruch 1 gegebene Bedeutung haben.

3. Die 5-(Pyridyl-2-oxy)-2-nitrobenzoesäurederivate gemäß Anspruch 1, entsprechend der Formel Ia

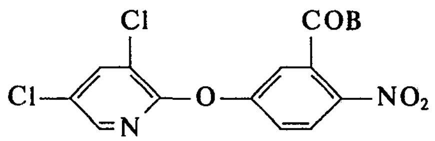

(Ia)

worin B die in Anspruch 1 gegebene Bedeutung hat.

4. Die 5-(Pyridyl-2-oxy)-2-nitrobenzoesäurederivate gemäß Anspruch 1, entsprechend der Formel Ib

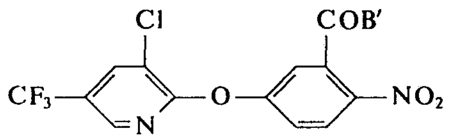

(Ib)

in welcher B' einen Rest $-OR_1$, $-SR_2$, $-NR_3R_4$ oder $-ON{=}C(R_5)_2$ bedeutet, worin $R_1$ einen substituierten $C_1$—$C_{18}$ Alkylrest, einen $C_3$—$C_{10}$ Alkenylrest, einen $C_3$—$C_8$ Alkinylrest, einen $C_3$—$C_{12}$ Cycloalkylrest, einen unsubstituierten oder durch Halogen, $C_1$—$C_4$ Alkyl, $C_1$—$C_4$ Alkoxy, $C_1$—$C_4$ Alkylthio, $-NO_2$, $-CF_3$, $-COOH$, $-CN$, $-OH$, $-SO_3H$, $-NH_2$, $-NH(C_1$—$C_4$ Alkyl) oder $-N(C_1$—$C_4$ Alkyl$)_2$ ein- oder mehrfach substituierten Phenylrest bedeutet und $R_2$, $R_3$, $R_4$ und $R_5$ die im Anspruch 1 gegebene Bedeutung haben, mit der Maßgabe, daß $R_1$ nicht für einen $C_1$—$C_{12}$ Alkylrest, welcher gegebenenfalls durch $C_1$—$C_8$ Alkoxy substituiert ist, und $R_3$ und $R_4$ nicht je für Wasserstoff, einen $C_1$—$C_4$ Alkylrest, einen $C_3$—$C_5$ Alkenylrest oder eines davon auch für einen $C_1$—$C_4$ Alkylsulfonylrest stehen.

5. Die 5-(Pyridyl-2-oxy)-2-nitrobenzoesäurederivate gemäß Anspruch 1 der Formel Ic

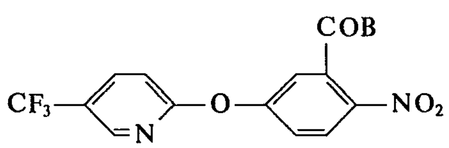

(Ic)

in denen B die im Anspruch 1 gegebene Bedeutung hat.

6. Die 5-(Pyridyl-2-oxy)-2-nitrobenzoesäurederivate gemäß Anspruch 1, in denen X und Y Chlor oder X Trifluormethyl und Y Wasserstoff oder Chlor und A den Rest $-COB$ bedeuten, worin B für Hydroxyl, Alkoxy mit 1 bis 18 Kohlenstoffatomen, Cyanalkoxy mit 1 bis 4 Kohlenstoffatomen, Alkoxycarbonylalkoxy mit 3 bis 18 Kohlenstoffatomen, Di-($C_1$—$C_4$ Alkyl)-amino-$C_1$—$C_4$-alkoxy, Alkenyloxy mit 3 bis 10 Kohlenstoffatomen, Alkinyloxy mit 3 bis 8 Kohlenstoffatomen, Alkylthio mit 1 bis 18 Kohlenstoffatomen, Alkoxycarbonylalkylthio mit 3 bis 18 Kohlenstoffatomen, Alkenylthio mit 3 bis 10 Kohlenstoffatomen, Alkylamino mit 1 bis 4 Kohlenstoffatomen, Alkoxyalkylamino mit 2 bis 8 Kohlenstoffatomen, Alkoxycarbonylalkylamino mit 3 bis 8 Kohlenstoffatomen oder N-$C_1$—$C_4$-Alkoxy-N-$C_1$—$C_4$-alkylamino steht, mit der Maßgabe, daß, wenn X Trifluormethyl und zugleich Y Wasserstoff oder Chlor bedeutet, A nicht für einen Rest $-COB$, in welchem B Hydroxyl, Alkoxy mit 1 bis 12 Kohlenstoffatomen oder Alkylamino mit 1 bis 4 Kohlenstoffatomen bedeutet, steht.

7. Die Verbindungen der Formel I gemäß Anspruch 1 in denen X Chlor und Y Wasserstoff bedeuten während A die in Anspruch 1 gegebene Bedeutung hat.

8. 5-(3-Chlor-5-trifluormethylpyridyl-2-oxy)-2-nitrobenzoesäureallylester gemäß Anspruch 1.

9. 5-(3-Chlor-5-trifluormethylpyridyl-2-oxy)-2-nitrobenzoesäure-(äthoxycarbonyläth-1'-yl)-ester gemäß Anspruch 1.

10. 5-(3-Chlor-5-trifluormethylpyridyl-2-oxy)-2-nitrobenzoesäure-(methoxycarbonylmethylthio)-ester gemäß Anspruch 1.

11. 5-(3,5-Dichlorpyridyl-2-oxy)-2-nitrobenzoesäuremethylester gemäß Anspruch 1.

12. 5-(3,5-Dichlorpyridyl-2-oxy)-2-nitrobenzoesäureäthylamid gemäß Anspruch 1.

13. 5-(5-Chlorpyridyl-2-oxy)-2-nitrobenzoesäure, gemäß Anspruch 1.

14. 5-(3-Chlor-5-trifluormethylpyridyl-2-oxy)-2-nitrobenzoesäure-(2'-dimethylaminoäthyl)-ester gemäß Anspruch 1.

15. 5-(3-Chlor-5-trifluormethylpyridyl-2'-oxy)-2-nitrobenzoesäure-propinyl-ester gemäß An-Anspruch 1.

16. 5-(3-Chlor-5-trifluormethylpyridyl-2-oxy)-2-nitrobenzoesäure-(2'-chloräthyl)-ester gemäß

Anspruch 1.

17. 5-(3-Chlor-5-trifluormethylpyridyl-2-oxy)-2-nitro-thiobenzoesäuremethylester gemäß Anspruch 1.

18. 5-(3-Chlor-5-trifluormethylpyridyl-2-oxy)-2-nitrobenzoesäure-(4'-bromphenyl)-ester gemäß Anspruch 1.

19. Verfahren zur Herstellung der 5-(Pyridyl-2-oxy)-2-nitrobenzoesäurederivate der Formel I, Anspruch 1, dadurch gekennzeichnet, daß man in einem aprotischen Lösungsmittel, in Gegenwart der säurebindenden Menge einer Base, ein 2-Halogenpyridin der Formel II

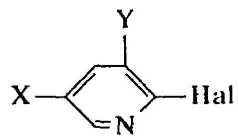

(II)

worin Hal ein Halogenatom bedeutet und X und Y die bezüglich Formel I, Anspruch 1 gegebene Bedeutung haben, mit einem Derivat der 5-Hydroxy-2-nitrobenzoesäure der Formel III umsetzt,

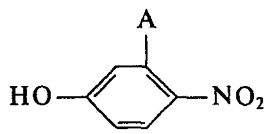

(III)

worin A einen bezüglich Formel I, Anspruch 1 definierten Rest darstellt.

20. Verfahren zur Herstellung der 5-(Pyridyl-2-oxy)-2-nitrobenzoesäurederivate der Formel I, Anspruch 1, dadurch gekennzeichnet, daß man ein 5-(Pyridyl-2-oxy)-benzoesäurederivat der Formel V,

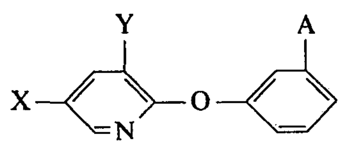

(V)

worin A, X und Y die bezüglich Formel I, Anspruch 1 gegebene Bedeutung haben, mit Salpetersäure oder einem Salpetersäure enthaltenden Gemisch nitriert.

21. Herbizides Mittel, dadurch gekennzeichnet, daß es als Wirkstoff mindestens ein 5-(Pyridyl-2-oxy)-2-nitrobenzoesäurederivat der Formel I, Anspruch 1 enthält.

22. Die Verwendung der Verbindungen der Formel I, Anspruch 1 zur selektiven Bekämpfung von Unkräutern in Kulturen von Nutzpflanzen.

23. Die Verwendung der Verbindungen der Formel I, Anspruch 1 zur selektiven Bekämpfung von Unkräutern in Sojakulturen.

24. Die Verwendung der Verbindungen der Formel I, Anspruch 1 zur selektiven Bekämpfung von Unkräutern in Getreidekulturen.

25. Die Verwendung der Verbindungen der Formel I, Anspruch 1 zur selektiven Bekämpfung von Unkräutern in Reiskulturen.

## Claims

1. A 5-(pyridyl-2-oxy)-2-nitrobenzoic acid derivate of the formula I

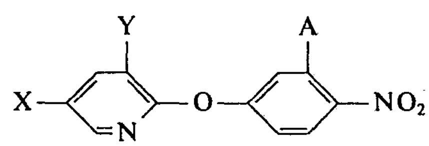

(I)

wherein X is halogen or a halomethyl group, Y is hydrogen, halogen or a halomethyl group, A is the cyano group, or a $-COB$ radical, B is a $-OR_1$, $-SR_2$, $-NR_3R_4$ or $-ON{=}C(R_5)_2$ radical, wherein $R_1$ is hydrogen or the cation of a base

$$\left[\frac{1}{n}M\right]^{n\oplus},$$

wherein M is an alkali metal cation or an alkaline earth metal cation or an iron, copper zinc or

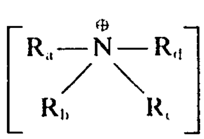

cation, n as 1, 2 or 3 corresponds to the valency of the cation, and each of $R_a$, $R_b$, $R_c$ and $R_d$ independently is hydrogen, benzyl, or a $C_1$—$C_4$alkyl radical which may be substituted by $-OH$, $-NH_2$ or $C_1$—$C_4$-alkoxy ; $R_1$ and $R_2$ are a $C_1$—$C_{18}$alkyl radical which is unsubstituted or substituted by halogen, nitro, cyano, $C_1$—$C_8$alkoxy, $C_2$—$C_8$alkoxyalkoxy, oxirane, $C_3$—$C_6$alkenyloxy, $C_1$—$C_8$alkylthio, $C_2$—$C_8$alkanoyl, $C_2$—$C_8$acyloxy, $C_2$—$C_8$alkoxycarbonyl, carbamoyl, $C_1$—$C_4$alkylamino, bis($C_1$—$C_4$alkyl)amino, $C_3$—$C_4$-alkenylamino, bis($C_3$—$C_4$alkenyl)amino, $C_3$—$C_4$alkynylamino, bis($C_3$—$C_4$alkynyl)amino, tri($C_1$—$C_4$alkyl)-amino, $C_3$—$C_8$cycloalkyl, $C_3$—$C_8$cycloalkenyl, or also by a phenyl, phenoxy or 5- or 6-membered heterocyclic radical containing 1 to 3 hetero atoms selected from the group consisting of $-O-$, $-S-$ and/or $-N-$ and unsubstituted or substituted by one or more identical or different members selected from the group consisting of halogen, $C_1$—$C_4$alkyl or $C_1$—$C_4$alkoxy; or is a $C_3$—$C_{10}$alkenyl radical which is unsubstituted or substituted by 1 to 4 halogen atoms or monosubstituted by phenyl or methoxycarbonyl; or is a $C_3$—$C_8$alkynyl radical; a $C_3$—$C_{12}$cycloalkyl radical which is unsubstituted or substituted by halogen or $C_1$—$C_4$alkyl; a $C_3$—$C_8$cycloalkenyl radical, a phenyl radical which is unsubstituted or substituted by one or more identical or different members selected from the group consisting of halogen, $C_1$—$C_4$alkyl, $C_1$— $C_4$alkoxy, $C_1$—$C_4$alkylthio, $-NO_2$, $-CF_3$, $-COOH$, $-CN$, $-OH$, $-SO_3H$, $-NH_2$ or $-NH(C_1$—$C_4$alkyl) or $-N(C_1$—$C_4$alkyl$)_2$; or is a 5- or 6-membered heterocyclic ring containing 1 to 3 hetero atoms selected from the group consisting of $-O-$, $-S-$ and/or $-N-$; and each of $R_3$ and $R_4$ is hydrogen, a $C_1$—$C_8$-alkyl radical which is unsubstituted or substituted by $C_1$—$C_4$alkoxy, hydroxy, halogen or $C_2$—$C_8$alkoxy-carbonyl, a $C_3$—$C_5$alkenyl radical, a $C_3$—$C_5$alkynyl radical, a $C_3$—$C_7$cycloalkyl radical, a pyridyl radical, or an unsubstituted or chlorine-substituted phenyl radical, or one of $R_3$ and $R_4$ is also a $C_1$—$C_4$alkoxy or $C_1$—$C_4$alkylsulfonyl radical, or $R_3$ and $R_4$, together with the nitrogen atom to which they are attached, are also a 5- or 6-membered heterocyclic ring system which can contain an additional oxygen or sulfur atom or an imino group which is unsubstituted or substituted by $C_1$—$C_3$alkyl, and each $R_5$ is $C_1$—$C_4$-alkyl or both together form a $C_3$—$C_5$alkylene chain, with the proviso that, if X is fluorine, chlorine, bromine, iodine, difluormethyl, chlorodifluoromethyl or trifluoromethyl and simultaneously Y is hydrogen, fluorine, chlorine, bromine, iodine, difluoromethyl, chlorodifluoromethyl or trifluoromethyl, and at least one of the substituents X and Y is difluoromethyl, chlorodifluoromethyl or trifluoromethyl, A may not be a $-COB$ radical, wherein B is $-OR_1$ or $-NR_3R_4$, wherein $R_1$ is hydrogen, the cation of a base

$$\left[\frac{1}{n}M\right]^{n\oplus},$$

where M and n are as defined above, or a $C_1$—$C_{12}$alkyl radical which is unsubstituted or substituted by $C_1$—$C_8$alkoxy, and each of $R_3$ and $R_4$ is hydrogen, a $C_1$—$C_8$alkyl radical, a $C_3$—$C_5$alkenyl radical, or one of $R_3$ and $R_4$ is also a $C_1$—$C_4$alkylsulfonyl radical.

2. A 5-(pyridyl-2-oxy)-2-nitrobenzoic acid derivative according to claim 1, wherein A is the COB radical and X, Y and B are as defined in claim 1.

3. A 5-(pyridyl-2-oxy)-2-nitrobenzoic acid derivative according to claim 1 of the formula Ia

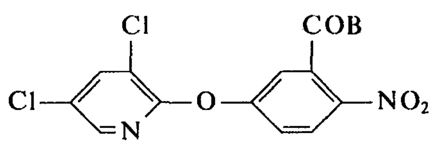

(Ia)

wherein B is as defined in claim 1.

4. A 5-(pyridyl-2-oxy)-2-nitrobenzoic acid derivative according to claim 1 of the formula Ib

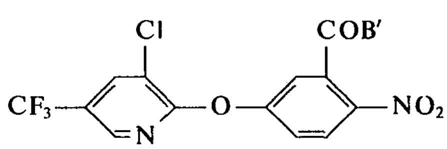

(Ib)

wherein B′ is a $-OR_1$, $-SR_2$, $-NR_3R_4$ or $-ON{=}C(R_5)_2$ radical, wherein $R_1$ is a substituted $C_1$—$C_{18}$alkyl radical, a $C_3$—$C_{10}$alkenyl radical, a $C_3$—$C_8$alkynyl radical, a $C_3$—$C_{12}$cycloalkyl radical, a phenyl radical which is unsubstituted or substituted by one more identical or different members selected from the group consisting of halogen, $C_1$—$C_4$alkyl, $C_1$—$C_4$alkoxy, $C_1$—$C_4$alkylthio, $-NO_2$, $-CF_3$, $-COOH$, $-CN$, $-OH$, $-SO_3H$, $-NH_2$, $-NH(C_1$—$C_4$alkyl) or $-N(C_1$—$C_4$alkyl$)_2$, and $R_2$, $R_3$, $R_4$ and $R_5$ are as defined in claim 1, with the proviso that $R_1$ is not a $C_1$—$C_{12}$alkyl radical which may be substituted by $C_1$—$C_8$alkoxy, and each of $R_3$ and $R_4$ is not hydrogen, a $C_1$—$C_8$alkyl radical, a $C_3$—$C_5$alkenyl radical, or one of $R_3$ and $R_4$

is also not a $C_1$—$C_4$alkylsulfonyl radical.

5. A 5-(pyridyl-2-oxy)-2-nitrobenzoic acid derivative according to claim 1 of the formula Ic

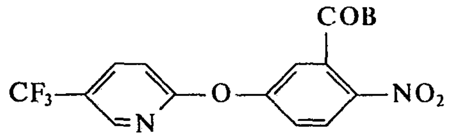

(Ic)

wherein B is as defined in claim 1.

6. A 5-(pyridyl-2-oxy)-2-nitrobenzoic acid derivative according to claim 1, wherein X and Y are chlorine or X is trifluoromethyl and Y is hydrogen or chlorine, and A is the —COB radical, wherein B is hydroxy, alkoxy of 1 to 18 carbon atoms, cyanoalkoxy of 1 to 4 carbon atoms, alkoxycarbonylalkoxy of 3 to 18 carbons, di($C_1$—$C_4$alkyl)amino-$C_1$—$C_4$alkoxy, alkenyloxy of 3 to 10 carbon atoms, alkynyloxy of 3 to 8 carbon atoms, alkylthio of 1 to 18 carbon atoms, alkoxycarbonylalkylthio of 3 to 18 carbon atoms, alkenylthio of 3 to 10 carbon atoms, alkylamino of 1 to 4 carbon atoms, alkoxyalkylamino of 2 to 8 carbon atoms, alkoxycarbonylalkylamino of 3 to 8 carbon atoms or N-$C_1$—$C_4$alkoxy-N-$C_1$—$C_4$alkylamino, with the proviso that, if X is trifluoromethyl and simultaneously Y is hydrogen or chlorine, A is not a —COB radical in which B is hydroxy, alkoxy of 1 to 12 carbon atoms or alkylamino of 1 to 4 carbon atoms.

7. A compound of the formula I according to claim 1, wherein X is chlorine and Y is hydrogen, and A is as defined in claim 1.

8. 5-(3-Chloro-5-trifluoromethylpyridyl-2-oxy)-2-nitrobenzoic acid allyl ester according to claim 1.

9. 5-(3-Chloro-5-trifluoromethylpyridyl-2-oxy)-2-nitrobenzoic acid (ethoxycarbonyleth-1'-yl) ester according to claim 1.

10. 5-(3-Chloro-5-trifluoromethylpyridyl-2-oxy)-2-nitrobenzoic acid (methoxycarbonylmethylthio) ester according to claim 1.

11. 5-(3,5-Dichloropyridyl-2-oxy)-2-nitrobenzoic acid methyl ester according to claim 1.

12. 5-(3,5-Dichloropyridyl-2-oxy)-2-nitrobenzoic acid ethylamide according to claim 1.

13. 5-(5-Chloropyridyl-2-oxy)-2-nitrobenzoic acid according to claim 1.

14. 5-(3-Chloro-5-trifluoromethylpyridyl-2-oxy)-2-nitrobenzoic acid (2'-dimethylaminoethyl) ester according to claim 1.

15. 5-(3-Chloro-5-trifluoromethylpyridyl-2-oxy)-2-nitrobenzoic acid propynyl ester according to claim 1.

16. 5-(3-Chloro-5-trifluoromethylpyridyl-2-oxy)-2-nitrobenzoic acid (2'-chloroethyl) ester according to claim 1.

17. 5-(3-Chloro-5-trifluoromethylpyridyl-2-oxy)-2-nitrothiobenzoic acid methyl ester according to claim 1.

18. 5-(3-Chloro-5-trifluoromethylpyridyl-2-oxy)-2-nitrobenzoic acid (4'-bromophenyl) ester according to claim 1.

19. A process for the preparation of a 5-(pyridyl-2-oxy)-2-nitrobenzoic acid derivative of the formula I according to claim 1, which process comprises reacting a 2-halopyridine of the formula II

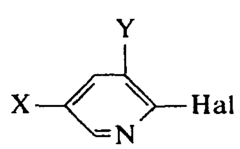

(II)

wherein Hal is a halogen atom and X and Y are as defined for formula I in claim 1, in an aprotic solvent and in the presence of a base which acts as acid acceptor, with a 5-hydroxy-2-nitrobenzoic acid derivative of the formula III

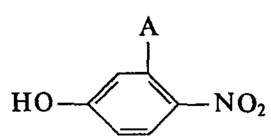

(III)

wherein A is a radical as defined under formula I in claim 1.

20. A process for the production of a 5-(pyridyl-2-oxy)-2-nitrobenzoic acid derivative of the formula I according to claim 1, which process comprises nitrating a 5-(pyridyl-2-oxy)-benzoic acid derivative of the formula V

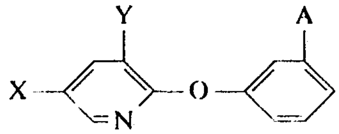

(V)

wherein A, X and Y are as defined for formula I in claim 1, with nitric acid or with a mixture containing nitric acid.

21. A herbicidal composition which contains, as active ingredient, at least one 5-(pyridyl-2-oxy)-2-nitrobenzoic acid derivative of the formula I according to claim 1.

22. A method of selectively controlling weeds in crops of useful plants, which comprises the use of a compound of formula I as defined in claim 1.

23. A method of selectively controlling weeds in soybean crops, which comprises the use of a compound of formula I as claimed in claim 1.

24. A method of selectively controlling weeds in crops of cereals, which comprises the use of a compound of formula I as claimed in claim 1.

25. A method of selectively controlling weeds in rice crops, which comprises the use of a compound of formula I as claimed in claim 1.

**Revendications**

1. Dérivés de l'acide 5-(pyridyl-2-oxy)-2-nitrobenzoïque de formule I

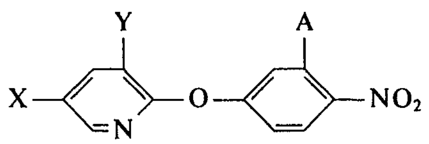

(I)

dans laquelle X représente un halogène ou un groupe halogénométhyle, Y représente l'hydrogène, un halogène ou un groupe halogénométhyle, A représente le groupe cyano ou un reste $-COB$, B représente un reste $-OR_1$, $-SR_2$, $-NR_3R_4$ ou $-ON{=}C(R_5)_2$, $R_1$ représente l'hydrogène ou le cation d'une base

$$\left[\frac{1}{n} M\right]^{n\oplus}$$

dans lequel M est un cation alcalin, alcalino-terreux ou un cation Fe, Cu, Zn ou

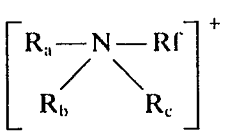

n est un nombre entier égal à 1, 2 ou 3 et qui correspond à la valence du cation, $R_a$, $R_b$, $R_c$ et $R_d$ représentant chacun, indépendamment les uns des autres, l'hydrogène, un groupe benzyle ou un groupe alkyle éventuellement substitué par des groupes $-OH$, $-NH_2$ ou alcoxy en $C_1$—$C_4$, $R_1$ et $R_2$ représentent un groupe alkyle en $C_1$—$C_{18}$ non substitué ou éventuellement substitué par des halogènes, des groupes oxiranne, nitro, cyano, alcoxy en $C_1$—$C_8$, alcoxyalcoxy en $C_2$—$C_8$, alcényloxy en $C_3$—$C_6$, alkylthio en $C_1$—$C_8$, alcanoyle en $C_2$—$C_8$, acyloxy en $C_2$—$C_8$, alcoxycarbonyle en $C_2$—$C_8$, carbamoyle, alkylamino en $C_1$—$C_4$, bis-(alkyle en $C_1$—$C_4$)-amino, alcénylamino en $C_3$—$C_4$, bis-(alcényle en $C_3$—$C_4$)-amino, alcynylamino en $C_3$—$C_4$, bis-(alcynyle en $C_3$—$C_4$)-amino, tri-(alkyle en $C_1$—$C_4$)-amino, cycloalkyle en $C_3$—$C_8$, cycloalcényle en $C_3$—$C_8$ ou également le cas échéant par un reste phényle, phénoxy ou hétérocyclique à 5—6 chaînons contenant un à trois hétéroatomes du groupe de l'oxygène, du soufre et/ou de l'azote, non substitué ou substitué une ou plusieurs fois par des halogènes, des groupes alkyle en $C_1$—$C_4$, alcoxy en $C_1$—$C_4$, un reste alcényle en $C_3$—$C_{10}$ non substitué ou substitué une à quatre fois par des halogènes ou par un groupe phényle ou méthoxycarbonyle, un reste alcynyle en $C_3$—$C_8$, un reste cycloalkyle en $C_3$—$C_{12}$ éventuellement substitué par des halogènes ou des groupes alkyle en $C_1$—$C_4$, un reste cycloalcényle en $C_3$—$C_8$, un reste phényle non substitué ou mono- ou polysubstitué par des halogènes, des groupes alkyle en $C_1$—$C_4$, alcoxy en $C_1$—$C_4$, alkylthio en $C_1$—$C_4$, $-NO_2$, $-CF_3$, $-COOH$, $-CN$, $-OH$, $-SO_3H$, $-NH_2$ ou $-NH$(alkyle en $C_1$—$C_4$)- ou $-N$(alkyle en $C_1$—$C_4)_2$, un reste hétérocyclique à 5 ou 6 chaînons contenant un à trois hétéroatomes du groupe de l'oxygène, du soufre et/ou de l'azote, $R_3$ et $R_4$ représentent chacun l'hydrogène, un groupe alkyle en $C_1$—$C_8$ éventuellement substitué par des groupes alcoxy en $C_1$—$C_4$, hydroxy, des halogènes ou des groupes alcoxycarbonyle en $C_2$—$C_8$, un reste alcényle en $C_3$—$C_5$, un reste alcynyle en $C_3$—$C_5$, un reste cycloalkyle en $C_3$—$C_7$, un reste pyridyle ou un reste phényle éventuellement substitué par le chlore ou bien l'un de ces symboles représente un groupe alcoxy en

$C_1$—$C_4$ ou alkyle en $C_1$—$C_4$-sulfonyle, $R_3$ et $R_4$ forment ensemble et avec l'atome d'azote auquel ils sont reliés également un hétérocycle à 5—6 chaînons qui peut encore contenir un atome d'oxygène ou de soufre ou un groupe imino éventuellement substitué par un groupe alkyle en $C_1$—$C_3$, chacun des symboles $R_5$ représente un groupe alkyle en $C_1$—$C_4$ ou bien les symboles $R_5$ représentent ensemble une chaîne alkylène en $C_3$—$C_5$, avec les restrictions que lorsque X représente le fluor, le chlore, le brome, l'iode, un groupe difluoro-, chlorodifluoro- ou trifluorométhyle et qu'en même temps Y représente l'hydrogène, le fluor, le chlore, le brome, l'iode, un groupe difluoro, chlorodifluoro- ou trifluorométhyle et qu'au moins un des symboles X et Y représente un groupe difluorochlorodifluoro- ou trifluorométhyle, A ne peut représenter un reste —COB dans lequel B représente —$OR_1$ ou —$NR_3R_4$ dans lequel $R_1$ représente l'hydrogène, le cation d'une base

$$\left[\frac{1}{n}M\right]^{n\oplus},$$

M et n ayant les significations indiquées ci-dessus, ou un reste alkyle en $C_1$—$C_{12}$ éventuellement substitué par un groupe alcoxy en $C_1$—$C_8$, et $R_3$ et $R_4$ représentent chacun l'hydrogène, un groupe alkyle en $C_1$—$C_8$, alcényle en $C_3$—$C_5$ ou bien l'un de ces symboles également un reste alkylsulfonyle en $C_1$—$C_4$.

2. Les dérivés de l'acide 5-(pyridyl-2-oxy)-2-nitrobenzoïque de la revendication 1, dans lesquels A représente le reste COB, X, Y et B ayant les significations indiquées dans la revendication 1.

3. Les dérivés de l'acide 5-(pyridyl-2-oxy)-2-nitrobenzoïque de la revendication 1, répondant à la formula Ia:

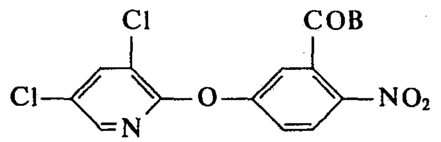

(Ia)

dans laquelle B a les significations indiquées dans la revendication 1.

4. Les dérivés de l'acide 5-(pyridyl-2-oxy)-2-nitrobenzoïque selon la revendication 1, qui répondent à la formule Ib:

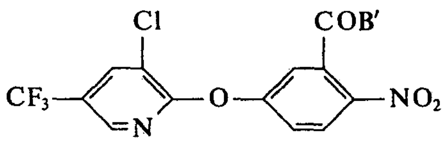

(Ib)

dans laquelle B' représente un groupe —$OR_1$, —$SR_2$, —$NR_3R_4$ ou —$ON{=}C(R_5)_2$ dans lesquels $R_1$ représente un groupe alkyle en $C_1$—$C_{18}$ substitué, un groupe alcényle en $C_3$—$C_{10}$, un groupe alcynyle en $C_3$—$C_8$, cycloakyle en $C_3$—$C_{12}$, un reste phényle non substitué ou mono- ou poly-substitué par des halogènes, des groupes alkyle en $C_1$—$C_4$, alcoxy en $C_1$—$C_4$, alkylthio en $C_1$—$C_4$, —$NO_2$, —$CF_3$, —COOH, —CN, —OH, —$SO_3H$, —$NH_2$, —NH(alkyle en $C_1$—$C_4$) ou —N(alkyle en $C_1$—$C_4$)$_2$, et $R_2$, $R_3$, $R_4$ et $R_5$ ont les significations indiquées dans la revendication 1, avec les restrictions que $R_1$ ne peut représenter un groupe alkyle en $C_1$—$C_{12}$ éventuellement substitué par un groupe alcoxy en $C_1$—$C_8$, et $R_3$ et $R_4$ ne peuvent représenter chacun l'hydrogène, un groupe alkyle en $C_1$—$C_8$, alcényle en $C_3$—$C_5$ ou bien l'un de ces symboles également un groupe alkylsulfonyle en $C_1$—$C_4$.

5. Les dérivés de l'acide 5-(pyridyl-2-oxy)-2-nitrobenzoïque de la revendication 1, répondant à la formule Ic:

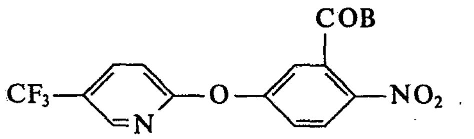

(Ic)

dans laquelle B a les significations indiquées dans la revendication 1.

6. Les dérivés de l'acide 5-(pyridyl-2-oxy)-2-nitrobenzoïque de la revendication 1, dans lesquels X et Y représentent le chlore ou bien X représente un groupe trifluorométhyle et Y l'hydrogène ou le chlore et A représente le reste —COB dans lequel B représente un groupe hydroxy, alcoxy en $C_1$—$C_{18}$, cyanalcoxy en $C_1$—$C_4$, alcoxycarbonylalcoxy en $C_3$—$C_{18}$, di-(alkyle en $C_1$—$C_4$)-amino-alcoxy en $C_1$—$C_4$, alcényloxy en $C_3$—$C_{10}$, alcynyloxy en $C_3$—$C_8$, alkylthio en $C_1$—$C_{18}$, alcoxycarbonylalkylthio en $C_3$—$C_{18}$, alcénylthio en $C_3$—$C_{10}$, alkylamino en $C_1$—$C_4$, alcoxyalkylamino en $C_2$—$C_8$, alcoxycarbonylalkylamino en $C_3$—$C_8$ ou N-(alcoxy en $C_1$—$C_4$)-N-(alkylamino en $C_1$—$C_4$), avec la restriction que lorsque X représente un groupe trifluorométhyle et en même temps Y l'hydrogène ou le chlore, A ne peut représenter un reste —COB dans lequel B représente un groupe hydroxy, alcoxy en $C_1$—$C_{12}$ ou alkylamino en $C_1$—$C_4$.

7. Les composé de formule I de la revendication 1, dans lesquels X représente le chlore et Y l'hydrogène, A ayant les significations indiquées dans la revendication 1.

8. Le 5-(3-chloro-5-trifluorométhylpyridyl-2-oxy)-2-nitrobenzoate d'allyle selon la revendication 1.

9. Le 5-(3-chloro-5-trifluorométhylpyridyl-2-oxy)-2-nitrobenzoate d'éthoxycarbonyléth-1'-yle selon la revendication 1.

10. Le méthoxycarbonylméthylthioester de l'acide 5-(3-chloro-5-trifluorométhylpyridyl-2-oxy)-2-nitrobenzoïque selon la revendication 1.

11. Le 5-(3,5-dichloropyridyl-2-oxy)-2-nitrobenzoate de méthyle selon la revendication 1.

12. L'éthylamide de l'acide 5-(3,5-dichloropyridyl-2-oxy)-2-nitrobenzoïque selon la revendication 1.

13. L'acide 5-(5-chloropyridyl-2-oxy)-2-nitrobenzoïque selon la revendication 1.

14. Le 5-(3-chloro-5-trifluorométhylpyridyl-2-oxy)-2-nitrobenzoate de 2'-diméthylaminoéthyle selon la revendication 1.

15. Le 5-(3-chloro-5-trifluorométhylpyridyl-2'-oxy)-2-nitrobenzoate de propynyle selon la revendication 1.

16. Le 5-(3-chloro-5-trifluorométhylpyridyl-2-oxy)-2-nitrobenzoate de 2'-chloréthyle selon la revendication 1.

17. Le 5-(3-chloro-5-trifluorométhylpyridyl-2-oxy)-2-nitro-thiobenzoate de méthyle selon la revendication 1.

18. Le 5-(3-chloro-5-trifluorométhylpyridyl-2-oxy)-2-nitrobenzoate de 4'-bromophényle selon la revendication 1.

19. Procédé de préparation des dérivés de l'acide 5-(pyridyl-2-oxy)-2-nitrobenzoïque de formule I de la revendication 1, caractérisé en ce que l'on fait réagir dans un solvant aprotonique, en présence de la quantité suffisante d'une base pour fixer les acides une 2-halogénopyridine de formule II

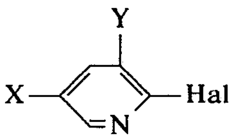

(II)

dans laquelle Hal représente un atome d'halogène et X et Y ont les significations indiquées en référence à la formule I dans la revendication 1, avec un dérivé de l'acide 5-hydroxy-2-nitrobenzoïque der formule III

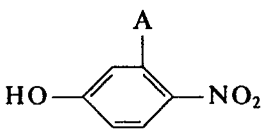

(III)

dans laquelle A représente un reste défini en référence à la formule I dans la revendication 1.

20. Procédé de préparation des dérivés de l'acide 5-(pyridyl-2-oxy)-2-nitrobenzoïque de formule I, revendication 1, caractérisé en ce que l'on nitre un dérivé de l'acide 5-(pyridyl-2-oxy)-benzoïque de formule V

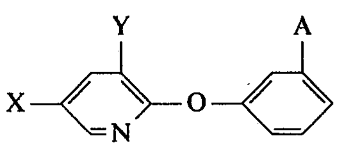

(V)

dans laquelle A, X et Y ont les significations indiquées en référence à la formule I, revendication 1, par de l'acide nitrique ou un mélange contenant de l'acide nitrique.

21. Produit herbicide caractérisé en ce qu'il contient en tant que substance active au moins un dérivé de l'acide 5-(pyridyl-2-oxy)-2-nitrobenzoïque de formule I, revendication 1.

22. L'utilisation des composés de formule I, revendication 1, pour la lutte sélective contre les mauvaises herbes dans les cultures de végétaux utiles.

23. L'utilisation des composés de formule I, revendication 1, pour la lutte sélective contre les mauvaises herbes dans les cultures de soja.

24. L'utilisation des composés de formule I, revendication 1, pour la lutte sélective contre les mauvaises herbes dans les cultures de céréales.

25. L'utilisation des composés de formule I, revendication 1, pour la lutte sélective contre les mauvaises herbes dans les cultures de riz.